(19) Europäisches Patentamt
European Patent Office
Office européen des brevets

(11) Veröffentlichungsnummer: **0 272 202 B1**

(12) **EUROPÄISCHE PATENTSCHRIFT**

(45) Veröffentlichungstag der Patentschrift: **01.07.92**

(51) Int. Cl.5: **C12P 41/00**, C12P 7/62

(21) Anmeldenummer: **87730146.5**

(22) Anmeldetag: **12.11.87**

(54) **Verfahren zur Herstellung von optisch aktiven Bicyclo [3.3.0] octandioncarbonsäureestern.**

(30) Priorität: **13.11.86 DE 3638760**

(43) Veröffentlichungstag der Anmeldung:
**22.06.88 Patentblatt 88/25**

(45) Bekanntmachung des Hinweises auf die Patenterteilung:
**01.07.92 Patentblatt 92/27**

(84) Benannte Vertragsstaaten:
**AT BE CH DE ES FR GB GR IT LI LU NL SE**

(56) Entgegenhaltungen:
**FR-A- 2 582 648**

**TETRAHEDRON, Band 42, Nr. 1, 1986, Seiten 435-444, Pergamon Press Ltd, GB; K. MORI et al.: "A new synthesis of (+)-6a-carbaprostaglandin I2 employing yeast reduction of a beta-keto ester derived from cis-bicyclo[3.3.0.]octane-3,7-dione as the key-step"**

**CHEM. PHARM. BULL., Band 33, Nr. 7, 1985, Seiten 2688-2696; K. KOJIMA et al.: "A convenient synthesis of (+)-9(0)-methanoprostacyclin"**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Petzold, Karl, Dr.**
**Flachsweg 10**
**W-1000 Berlin 38(DE)**
Erfinder: **Skuballa, Werner, Dr.**
**Olwenstrasse 25**
**W-1000 Berlin 28(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

### Stand der Technik

Optisch aktives 6a-Carba-prostacyclin und besonders einige davon abgeleitete Verbindungen besitzen als stabile Analoge des natürlichen Prostacyclins ($PGI_2$) einen hohen therapeutischen Nutzen [R.C. Nickolson, M.H. Town, H. Vorbrüggen: Prostacyclin-Analogs, Medicinal Research Reviews, Vol. 5, No. 1, S. 1-53 (1985)]. Die in dieser neueren Übersicht aufgeführten Synthesen sind lang und führen teilweise nur zu racemischen Carbacyclinen. Besonders aufwendig sind die Synthesen, die zu Carbacyclinen in der dem natürlichen $PGI_2$ entsprechenden absoluten Konfiguration führen. Das liegt daran, daß gut zugängliche, geeignete Ausgangsmaterialien achiral sind und die optische Aktivität erst im Syntheseverlauf in hierfür geeignete Zwischenstufen eingeführt werden muß.

Mehrere Synthesen gehen bereits von optisch aktiven 7α-Hydroxy-6β-hydroxymethyl-2-oxa-bicyclo-[3.3.0]octan-3-on-Derivaten aus. Damit ist zwar das Problem der Einführung der optischen Aktivität gelöst. Es müssen jedoch noch weitere vielstufige Synthesesequenzen für die Substitution der 2-Oxa-Funktion durch eine Methylengruppe durchgeführt werden, um zu Derivaten des 3α-Hydroxy-2β-hydroxymethyl-bicyclo[3.3.0]octan-7-ons zu gelangen, die sich erst für den Anbau der für die Carbacyclin-Analoga jeweils typischen α- und ω-Ketten eignen.

Eine neuere Publikation beschreibt die Verwendung von cis-Bicyclo[3.3.0]octan-3,7-dion-Derivaten zur Synthese optisch aktiver Carbacycline. Kojima et al. beschreiben in Chem. Pharm. Bull. 33, 2688 (1985) ein Verfahren, das die Trennung diastereomerer Salze der racemischen 7,7-Ethylendioxy-3α-hydroxy-cis-bicyclo[3.3.0]octan-2-carbonsäure einschließt.

Auch dieses Verfahren erfordert noch 7 Reaktionsschritte, um ausgehend von 3-Oxoglutarestern zum Startmaterial für Carbacyclin-Analoga zu gelangen. Zudem wird eine instabile β-Ketosäure-Zwischenstufe durchlaufen.

Für die Herstellung optisch aktiver Carbacyclin-Analoga, wie sie oben beschrieben werden, ist weiterhin kein Syntheseweg, der eine einfache Herstellung gestattet, bekannt.

Es wurde nun gefunden, daß sich die oben genannten prochiralen Dicarbonsäureester von Prostacyclin- und Carbacyclin-Zwischenstufen in sehr guten Ausbeuten enantioselektiv zu den Monocarbonsäureestern verseifen und decarboxylieren lassen, wenn man hierfür α-Chymotrypsin, verwendet.

Die Erfindung eignet sich besonders zur enzymatischen enantioselektiven Monoverseifung und Decarboxylierung der nachstehend genannten Prostacyclin- und Carbacyclin-Zwischenprodukte 1 - 4.

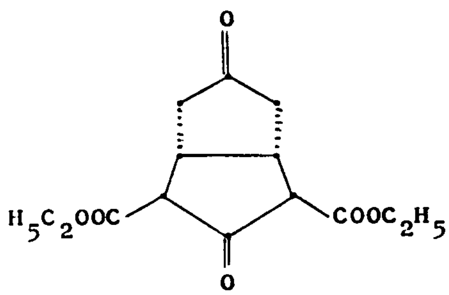

1

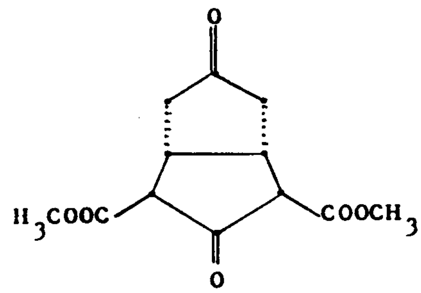

2

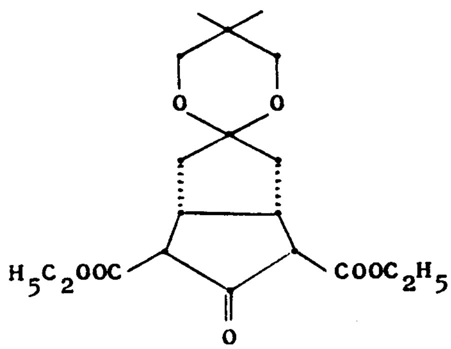

3

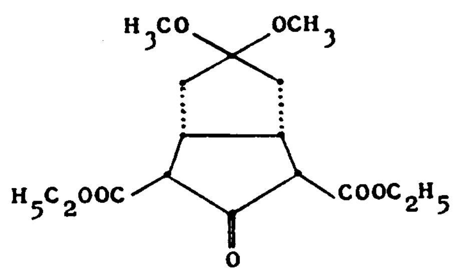

4

Die Erfindung betrifft somit ein Verfahren zur Herstellung von optisch aktiven Bicyclo[3.3.0]-octandioncarbonsäureestern der Formel I

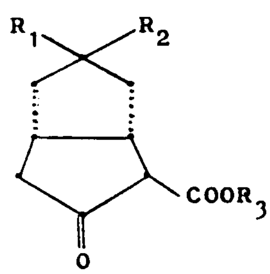

(I),

worin

| | |
|---|---|
| $R_1$ und $R_2$ | gemeinsam ein Sauerstoffatom oder den Rest -O-X-O- mit X in der Bedeutung einer gerad- oder verzweigtkettigen Alkylengruppe mit 1 - 7 C-Atomen oder |
| $R_1$ und $R_2$ | jeweils den Rest $OR_4$ mit $R_4$ als gerad- oder verzweigtkettigem Alkyl mit 1 - 7 C-Atomen und |
| $R_3$ | eine gerad- oder verzweigtkettige Alkylgruppe mit 1 - 10 C-Atomen darstellen. |

dadurch gekennzeichnet, daß man einen prochiralen Bicyclo[3.3.0]octandiondicarbonsäurediester der Formel II

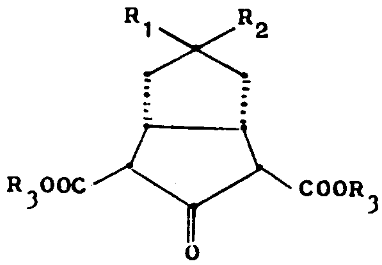

(II),

worin $R_1$, $R_2$ und $R_3$ die oben genannten Bedeutungen haben, enantioselektiv mit α-Chymotrypsin verseift und decarboxyliert.

Wenn X einen geradkettigen oder verzweigtkettigen Alkylenrest mit 1 - 7 C-Atomen bedeutet, so sind damit folgende Reste gemeint:
$-(CH_2)_n-$ mit n = 1-7 (Methylen, Ethylen, Tri-, Tetra-, Penta-, Hexa- und Heptamethylen), $-C(CH_3)_2-$, $-CH(CH_3)-$, $-CH(CH_3)-CH_2-$, $-C(CH_3)_2-CH_2-$, $-CH_2-CH(CH_3)-$, $CH_2-C(CH_3)_2$, $-CH_2-CH(CH_3)-CH_2-$, $-CH_2-C(CH_3)_2-CH_2-$, $-CH-(C_2H_5)-$, $-C(C_2H_5)_2-$, $-CH(C_2H_5)-CH_2-$, $-C(C_2H_5)_2-CH_2-$, $-CH_2-CH(C_2H_5)-$, $-CH_2-C(C_2H_5)_2-$, $-CH_2-CH(C_2H_5)-CH_2-$, $-CH_2-C(C_2H_5)_2-CH_2-$ usw.

$R_3$ und $R_4$ als geradkettige oder verzweigtkettige Alkylreste mit 1 - 10 bzw. 1 - 7 C-Atomen bedeuten Methyl, Ethyl, n-Propyl, Isopropyl, n-Butyl, Isobutyl, sek.-Butyl, tert.-Butyl, n-Pentyl, Isopentyl, sek.-Pentyl, Neopentyl, n-Hexyl, Isohexyl, n-Heptyl, Isoheptyl, Octyl, Nonyl, Decyl.

Das erfindungsgemäß verwendete α-Chymotrypsin kann sowohl in gelöster oder in suspendierter Form als auch immobilisiert, zum Beispiel an BrCN-aktivierter Sepharose oder an Oxiranacrylperlen, eingesetzt werden.

Die Ausgangsverbindung für das obige Verfahren sind bekannt oder können wie folgt hergestellt werden:

**Bezugsbeispiel 1**

2,4-Bisethoxycarbonyl-bicyclo[3.3.0]octan-3,7-dion

Zu einer Suspension von 82 g Kaliumcarbonat in 680 ml Äthanol fügt man bei 25 °C eine Lösung von 86 g Acetondicarbonsäurediäthylester und 59 g Acetoxycyclopentenon in 170 ml Äthanol und rührt 24 Stunden bei 25 °C. Man engt dann im Vakuum ein, versetzt mit Wasser und säuert mit 20 %iger Zitronensäurelösung auf pH4 an. Man extrahiert dreimal mit Methylenchlorid, wäscht die organische Phase dreimal mit Sole, trocknet über Natriumsulfat und dampft im Vakuum ein. Den Rückstand reinigt man durch Chromatographie an Kieselgel. Mit Essigester/Hexan eluiert man 70 g der Titelverbindung als farbloses Öl.
IR ($CHCl_3$): 3020, 2960, 1740, 1665, 1623, 1445 $cm^{-1}$.

**Bezugsbeispiel 2**

2,4-(Bismethoxycarbonyl)-bicyclo[3.3.0]octan-3,7-dion

Zu einer Mischung aus 240 ml Acetondicarbonsäuredimethylester und 120 g Diisopropylethylamin tropft man eine Mischung aus 60 g Acetoxycyclopentenon und 120 ml Acetondicarbonsäuredimethylester bei ca. 20 °C. Anschließend rührt man 20 Stunden bei Raumtemperatur, säuert mit 20 %iger Zitronensäure auf pH3-4 an und extrahiert dreimal mit je 300 ml Methylenchlorid. Die organische Phase wäscht man mit 100 ml 5 %iger Natriumbicarbonatlösung und mit Sole, trocknet über Natriumsulfat und dampft im Vakuum ein. Man destilliert den überschüssigen Acetondicarbonsäuredimethylester bei 0,07 mbar und 97 °C ab und reinigt den Destillationsrückstand durch Säulenchromatographie an Kieselgel. Mit Essigester/Hexan (3 + 2) eluiert man 39 g der Titelverbindung als farbloses Öl.
IR ($CHCl_3$): 3022, 2961, 1740, 1665, 1624, 1445 $cm^{-1}$.

**Bezugsbeispiel 3**

2,4-Bisethoxycarbonyl-7,7-(2,2-dimethyl-propylen-1,3-dioxy)-bicyclo[3.3.0]octan-3-on

Zu einer Lösung von 10 g 2,4-Bisethoxycarbonylbicyclo[3.3.0]octan-3,7-dion(Herstellung Bezugsbeispiel 1) in 165 ml Methylenchlorid fügt man bei 25 °C 83 mg p-Toluolsulfonsäure, 8,2 g 2,2-Dimethylpropan-1,3-diol und 4,3 g ortho-Ameisensäuretriäthylester. Man rührt 24 Stunden bei 25 °C, versetzt mit 500 ml Methylenchlorid und wäscht einmal mit 30 ml 5 %iger Natriumcarbonatlösung und dreimal mit Sole. Man trocknet über Natriumsulfat und dampft im Vakuum ein. Den Rückstand reinigt man durch Chromatographie an Kieselgel. Mit Essigester/Hexan (3+2) eluiert man 10,3 g der Titelverbindung als farbloses Öl.
IR ($CHCl_3$); 2955, 2883, 1735, 1665 $cm^{-1}$.

**Bezugsbeispiel 4**

2,4-Bisethoxycarbonyl-7,7-dimethoxy-bicyclo[3.3.0]octan-3-on

Zu einer Lösung von 9,6 g 2,4-Bisethoxycarbonylbicyclo[3.3.0]octan-3,7-dion (Herstellung Bezugsbeispiel 1) in 330 ml Methanol fügt man bei 25 °C 330 mg p-Toluolsulfonsäure und 1,6 ml Orthoameisensäuretrimethylester. Man rührt 3 Stunden bei 25 °C, versetzt mit 80 ml 5 %iger Natriumbicarbonatlösung, extrahiert dreimal mit je 300 ml Methylenchlorid, trocknet über Natriumsulfat und dampft im Vakuum ein. Den Rückstand reinigt man durch Chromatographie an Kieselgel. Mit Essigester/Hexan (1+1) erhält man 7 g der Titelverbindung als farbloses Öl.

Die nach dem erfindungsgemäßen Verfahren hergestellen Verbindungen der Formel I dienen zur Herstellung pharmakologisch wirksamer Protacyclin-Derivate.

Die Verbindungen der allgemeinen Formel I können zur Herstellung pharmakologisch wirksamer Carbacyclin-Derivate eingesetzt werden [siehe hierzu R.C. Nickolson, M.N. Town und H. Vorbrüggen. Medicinal Research Review 5, 1 (1985) und P.A. Aristoff in Advances in Prostaglandin, Thromboxane and Leukotriene Research, Vol. 15 (1985)].

Ausgehend von (1S, 2R, 5R)-2-Ethoxycarbonyl-7,7-(2,2-dimethyl-propylen-1,3-dioxy)-bicyclo[3.3.0]-octan-3-one gelangt man beispielsweise in einer mehrstufigen Synethese zum Wirkstoff Iloprost.

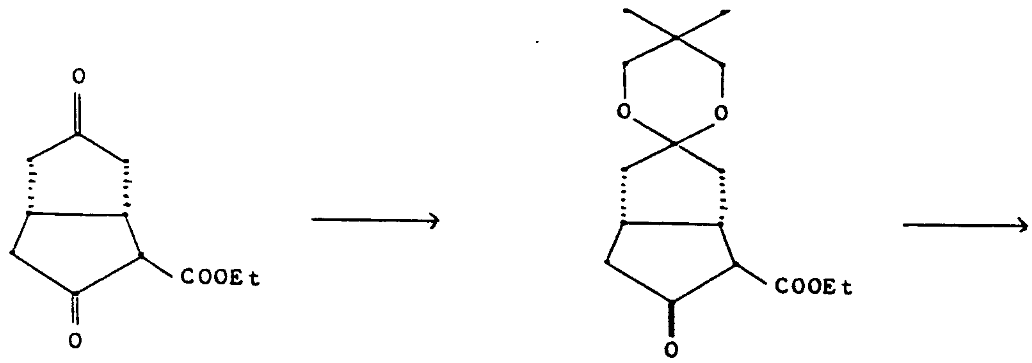

Ia

Ib

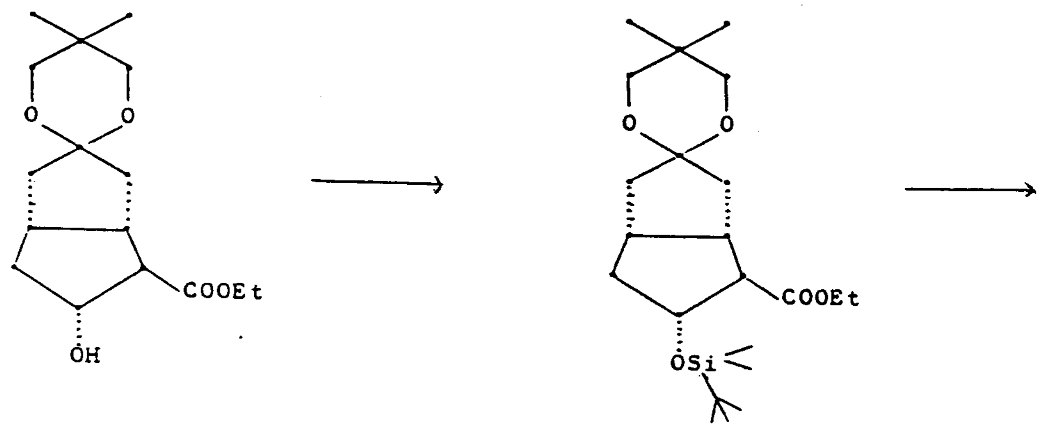

III

IV

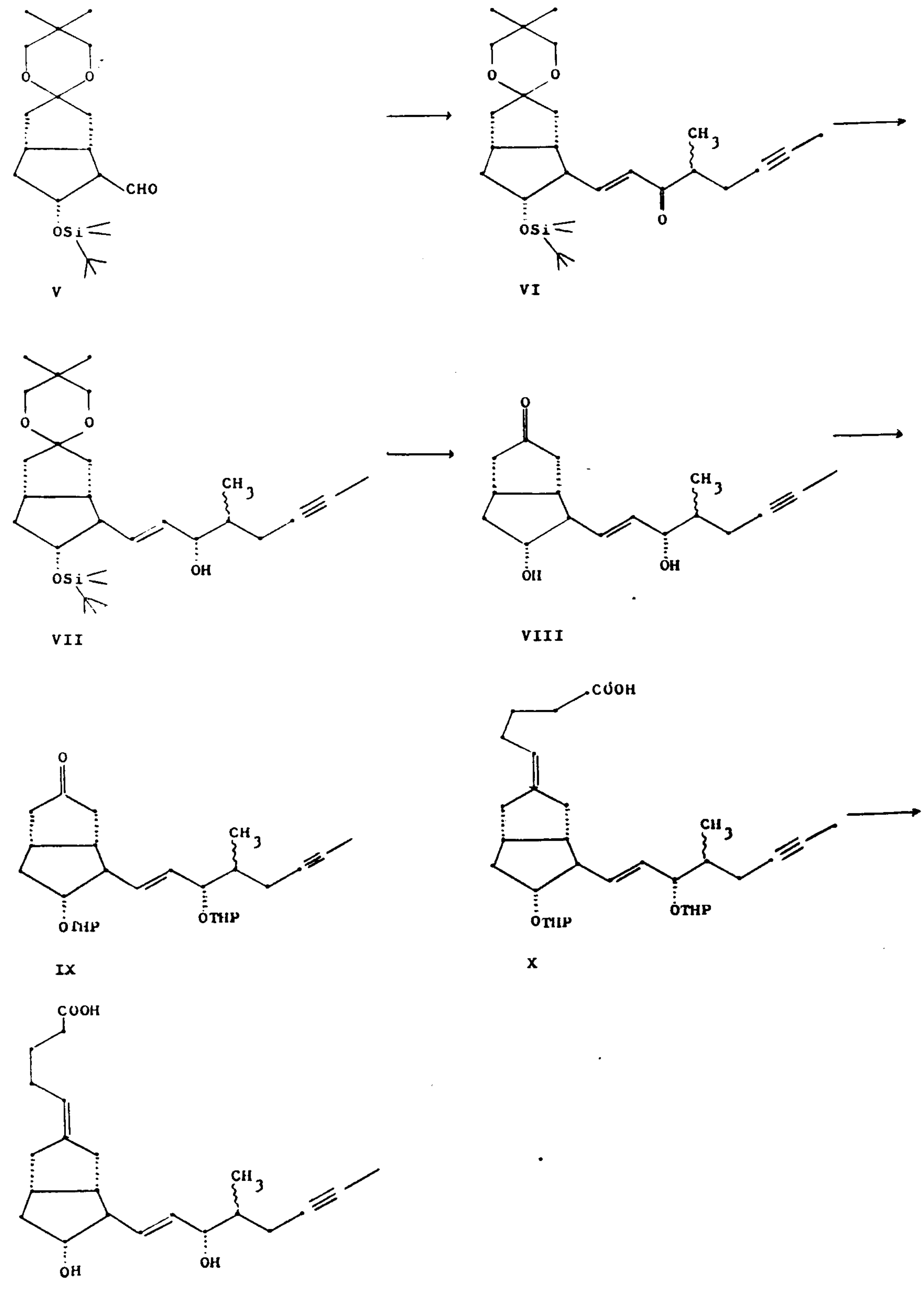

V

VI

VII

VIII

IX

X

Iloprost

Nach regioselektivem Schutz der Carbonylgruppe in Ia mit Ameisensäureäthylester in Gegenwart von 2,2-Dimethyl-1,3-diol und einer katalytischen Menge p-Toluolsulfonsäure wird Ib mit Natriumborhydrid in Äthanol zum Alkohol III reduziert. Silylätherbildung (IV) und anschließende Reduktion mit Diisobutylaluminiumhydrid in Toluol bei -70 °C liefert den Aldehyd V, der mit 3-Methyl-2-oxo-hept-5-in-phosphonsäuredimethylester und Natriumhydrid zum $\alpha,\beta$-ungesättigten Keton VI kondensiert wird. Reduktion des Ketons VI

zum Alkohol VII, anschließende Schutzgruppenspaltung zum Diol VIII und Tetrahydropyranylätherbildung liefert das Keton IX, welches nach Wittig-Reaktion mit dem Ylen aus 4-Carboxybutyltriphenylphosphoniumbromid und anschließender Schutzgruppenabspaltung mit wäßriger Essigsäure in das Carbacyclinderivat Iloprost überführt wird.

Die folgenden Ausführungsbeispiele dienen zur Erläuterung des erfindungsgemäßen Verfahrens.

**Beispiel 1**

10 g 2,4-Bisethoxycarbonyl-bicyclo[3.3.0]octan-3,7-dion werden in 40 ml Ethanol gelöst und mit 0,1 M Phosphatpuffer pH7 auf eine Volumen von 1000 ml verdünnt. Anschließend werden 5 g $\alpha$-Chymotrypsin aus Rinderpankreas (Aktivität 1150 U/mg, Fa. Chemie Pharmazie Commerz, Hamburg) zugegeben und 44 Stunden bei 30 °C auf einem Rotationsschüttler geschüttelt. Danach wird das Reaktionsgemisch dreimal mit Methylisobutylketon extrahiert, die Extrakte vereinigt und im Vakuum zur Trockne eingeengt. Der hinterbliebene ölige Rückstand (9,4 g) wird zur Reinigung über eine Kieselgelsäule mittels eines Lösungsmittelgradienten Hexan/Essigester chromatographiert. Nach dem Eindampfen der Hauptreaktion erhält man 6.68 g (89,7 % d. Th.) reines 2-Ethoxycarbonyl-bicyclo[3.3.0]octan-3,7-dion als helles farbloses Öl, das nicht kristallisiert.

Durch Vergleich des CD-Spektrums mit dem Spektrum einer auf anderem Wege dargestellten identischen Vergleichssubstanz ist ersichtlich, daß die erfindungsgemäß dargestellt Verbindung eine Enantiomerenreinheit von über 98 % aufweist.

**Beispiel 2**

1 g 2,4-Bismethoxycarbonyl-bicyclo[3.3.0]octan-3,7-dion wird in 20 ml Methanol gelöst und mit einer Lösung von 2,5 g $\alpha$-Chymotrypsin in 150 ml 0,1 M Phosphatpuffer pH7 vereinigt. Die Lösung wird nach 22stündigem Rühren bei 28 °C mit Methylisobutylketon extrahiert, der Extrakt im Vakuum eingeengt und der verbleibende Rückstand über eine Kieselgelsäule (Gradient Hexan/Essigester) chromatographiert. Man erhält 610 mg optisch reines 2-Methoxycarbonyl-bicyclo[3.3.0]octan-3,7-dion als ölige Flüssigkeit.

**Beispiel 3**

3 g 2,4-Bisethoxycarbonyl-7,7-dimethoxy-bicyclo[3.3.0]octan-3-on werden in 12 ml Ethanol gelöst und mit 288 ml 0,1 M Phosphatpuffer pH7 vereinigt. Nach Zugabe von 3 g $\alpha$-Chymotrypsin wird 30 Stunden auf einem Rotationsschüttler bei 30 °C geschüttelt. Anschließend wird die Reaktionslösung gefriergetrocknet und der verbliebene Rückstand mit Methylisobutylketon eluiert. Das Eluat wird wiederem zur Trockne gebracht und über eine Kieselgelsäule (Gradient Hexan/Essigester) chromatographiert. Nach dem Eindampfen der Hauptfraktion erhält man 1,64 g optisch reines 2-Ethoxycarbonyl-7,7-dimethoxy-bicyclo[3.3.0]octan-3-on in Form eines nicht kristallinierenden Öls.

## Patentansprüche

**1.** Verfahren zur Herstellung von optisch aktiven Bicyclo[3.3.0]octan dioncarbonsäureestern der Formel I

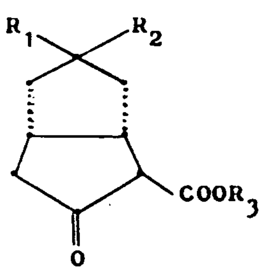

(I),

worin

| | |
|---|---|
| $R_1$ und $R_2$ | gemeinsam ein Sauerstoffatom oder den Rest -O-X-O- mit X in der Bedeutung einer gerad- oder verzweigtkettigen Alkylengruppe mit 1 - 7 C-Atomen oder |
| $R_1$ und $R_2$ | jeweils den Rest $OR_4$ mit $R_4$ als gerad- oder verzweigtkettigem Alkyl mit 1 - 7 C-Atomen und |

$R_3$ eine gerad- oder verzweigtkettige Alkylgruppe mit 1 - 10 C-Atomen darstellen.

dadurch gekennzeichnet, daß man einen prochiralen Bicyclo[3.3.0]octandiondicarbonsäurediester der Formel II

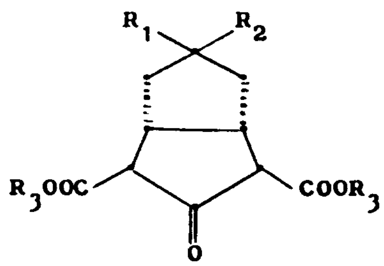

(II),

worin $R_1$, $R_2$ und $R_3$ die oben genannten Bedeutungen haben, enantioselektiv mit $\alpha$-Chymotrypsin verseift und decarboxyliert.

**Claims**

1. Process for the preparation of optically active bicyclo[3.3.0]octanedionecarboxylic acid esters of formula I

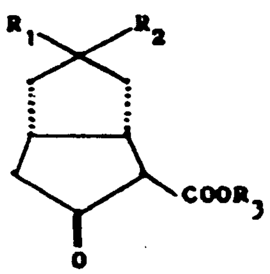

(I),

wherein

$R_1$ and $R_2$ together represent an oxygen atom or the radical -O-X-O- wherein X represents a straight-chain or branched alkylene group having from 1 to 7 carbon atoms, or

$R_1$ and $R_2$ each represents the radical $OR_4$ wherein $R_4$ represents a straight-chain or branched alkyl group having from 1 to 7 carbon atoms, and

$R_3$ represents a straight-chain or branched alkyl group having from 1 to 10 carbon atoms,

characterised in that a prochiral bicyclo[3.3.0]octanedionedicarboxylic acid diester of formula II

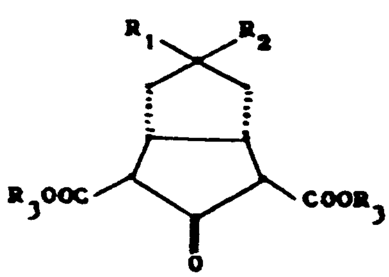

(II),

wherein $R_1$, $R_2$ and $R_3$ have the meanings given above, is enantioselectively hydrolysed and decarboxylated with $\alpha$-chymotrypsin.

**Revendications**

1. Procédé pour préparer des esters d'acides bicyclo[3.3.0]octane-dione-carboxyliques optiquement actifs

qui répondent à la formule I :

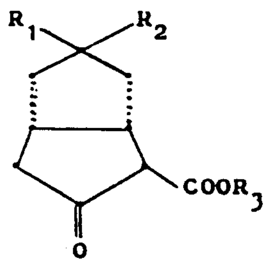

(I),

dans laquelle

- $R_1$ et $R_2$ forment ensemble un atome d'oxygène ou un radical -O-X-O- dont le symbole X représente un radical alkylène, linéaire ou ramifié, qui contient de 1 à 7 atomes de carbone, ou
- $R_1$ et $R_2$ représentent chacun un radical -OR4 dont le symbole R4 représente un radical alkyle, linéaire ou ramifié, qui contient de 1 à 7 atomes de carbone, et
- $R_3$ représente un radical alkyle, linéaire ou ramifié, qui contient de 1 à 10 atomes de carbone,

procédé caractérisé en ce qu'on saponifie et décarboxyle énantiosélectivement, à l'aide de l'α-chymotrypsine, un diester d'acide bicyclo[3.3.0]octane-dione-dicarboxylique prochiral répondant à la formule II :

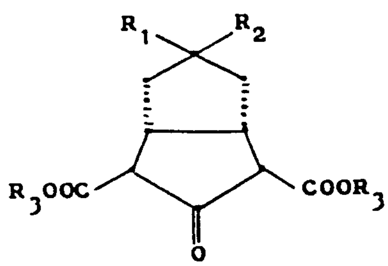

(II),

dans laquelle $R_1$, $R_2$ et $R_3$ ont les significations qui leur ont été données ci-dessus.